# EUROPEAN PATENT SPECIFICATION

(11) **EP 4 459 634 B1**
(45) Date of publication and mention of the grant of the patent: **12.03.2025**
(21) Application number: 23171642.4
(22) Date of filing: 04.05.2023
(51) Int. Cl.: G16H 50/00, G16H 50/20

(54) **COMPUTER IMPLEMENTED METHOD, COMPUTER SYSTEM AND COMPUTER PROGRAM PRODUCT FOR DETERMINING A MENOPAUSAL STATE**
COMPUTERIMPLEMENTIERTES VERFAHREN, COMPUTERSYSTEM UND COMPUTERPROGRAMMPRODUKT ZUR BESTIMMUNG EINES MENOPAUSALEN ZUSTANDS
PROCÉDÉ MIS EN UVRE PAR ORDINATEUR, SYSTÈME INFORMATIQUE ET PRODUIT DE PROGRAMME INFORMATIQUE POUR DÉTERMINER UN ÉTAT MÉNOPAUSIQUE

(43) Date of publication of application: 06.11.2024
(73) Proprietor: IdentifyHer Ltd., Dublin 2 D02P950 (IE)
(72) Inventor: O'Gorman, Donal, Slane, Co. Meath, C15XVY4 (IE); Kerr, Alish, Mornington, Co. Meath, A92VH6Y (IE); Davis, Heidi, Brannockstown, Kildare W91AY77 (IE)
(74) Representative: Ziebig Hengelhaupt Intellectual Property Attorneys Patentanwaltskanzlei PartGmbB

(56) References cited:
- EP-A1- 3 591 659
- WO-A1-2022/032121
- US-A1- 2015 320 588
- US-A1- 2019 282 178
- US-A1- 2021 169 343

## Description

The invention relates to a computer implemented method and a computer implemented system for determining menopausal state, and in particular, relates to such a method, system and computer program product that employs sensors measuring data and processing the measured data by means of a self-learning model.

In the prior art, the basic principles of analyzing data with self-learning models or similar analysis methods in a medical context is known. A variety of different sensors are used to capture biophysical data signals which are then analyzed using intelligent computing methods like artificial neural networks and the like.

In another example, so-called symptom checkers analyze static patient record data as well as provided symptoms from interactive chats with patients deriving probabilities of relevant diseases.

In another example, glucose measurement for diabetes patients also uses sensor technologies to continuously measure values of blood parameters that can be used for deriving predictions and recommendations for treatment and medication. In terms of menopause symptom diagnosis and treatment, mainly symptom treatment is known in prior art such as cooling devices, blankets, or special clothing. In another example, heart rate sequence data analysis is used for determining a hot flash using a sensor device.

EP3591659A1 describes a system for managing hot flashes and menopause symptoms using a sensor circuitry in combination with a logic circuitry having a predictive model that indicates a probability of a person having a hot flash.

Some menopausal symptoms, however, are in general unspecific or not unique to menopause. They may occur in the context of several pathological or non-pathological conditions. Although certain principles of detecting different symptoms that inter alia occur with menopause are known in prior art, one of the main challenges remains the clear identification of menopausal symptoms as determining a menopausal state.

Menopausal symptoms indicating a menopausal state occur with large time spans and provide the challenge of large variations of possible symptom characteristics. More than 40 different possible symptoms are known to be related to menopause and occur in different compositions, frequency and severity. Menopausal symptoms, in particular perimenopausal symptoms may include vasomotor symptoms like hot flashes and night sweat, insomnia, decreased cognitive performance, depressive states, migraine, weight gain, anxiety, joint aches, palpitation, vaginal dryness, urinary symptoms and low libido. Postmenopause may result in an increase of FSH level, change in blood lipid profile, increased glucose intolerance, bone demineralization, increased blood pressure, osteoporosis and osteoarthritis.

As menopausal symptoms, in particular perimenopausal symptoms overlap with stressful states or anxiety states and in addition strongly vary in terms of timely occurrence (larger period of time) and intensity, as well as in their characteristics as a combination of a variety of different particular single body symptoms. Automated prediction and detection accuracy remains a technical challenge. In particular, an accurate differentiation of hot flushes to other comparable events and activities is not yet sufficiently solved. For example, an increased mean heart rate as well as an increased mean temperature, both per se and combined may be indicative of a menopausal state. However, if they occur together, they may be also caused by other factors like exercising.

At the same time, medical staff and physicians expect a medical-grade accuracy and not just a certain probability of a menopausal symptom.

Document Tsiartas, Baker, Smith, Zambotti, "A novel Hot-Flash classification algorithm via multi-sensor features integration", EMBC, Oct 31-Nov 4 2021, PMID 34891695 discloses a hot flash classification algorithm based on multi-sensor features integration using commercial wearable wrist worn sensors, aggregating photoplethysmographic (PPG), motion, temperature and skin conductance data. Via a decision tree, hot flashes are recorded and assessed. Sensor information is computed using ±15 second windows. Skin conductance features are compared to ±2 minutes around a hot flash or 250 seconds prior to the hot flash and the current 30 seconds window. Temperature features are compared between prior and following 500 seconds of a hot flash. However, the teaching requires a large amount of computational resources and provides insufficiently accurate results.

Document US 2015/320588 A1 discloses a sleep environment control system which uses wearable technology with physiological sensors to predict when a person will have a hot flash and to proactively provide localized cooling or accelerated airflow for that person for a limited time to alleviate the adverse effects of that hot flash. In an example, a physiological sensor can be a body temperature sensor, skin conductance sensor, or EEG sensor. This system can reduce interruptions of a person's sleep due to hot flashes and improve their quality of life.

Document WO 2022/032121 A1 discloses systems, devices, and methods involving hot flash (HF) multi-sensor circuits. An example system includes a plurality of sensor circuits and processor circuitry. The sensor circuits obtain a plurality of sensor signals associated with the user. The processor circuitry extracts features from the plurality of sensor signals obtained by the plurality of sensor circuits, aligns the extracted features to a common time point, identifies a HF event for the user using a predictive data model indicative of probability of the HF event occurring for the user at a date and time and based on the aligned extracted features, and communicates a message indicative of the HF event to the user. Document US 2021/169343 A1 discloses a method of detecting the occurrence of a hot flash in an individual including obtaining heart rate sequence data for the individual for a predetermined period of time, wherein the heart rate sequence data is based on heartbeat data of the individual that is detected by a sensor unit worn by the individual, providing the heart rate sequence data to a computational model component, wherein the computational model component is structured and configured to examine the heart rate sequence data over time to determine a probability that the individual is experiencing a hot flash based on monitoring the heart rate sequence data for a pattern wherein heart rate decreases below a baseline range and then increases above the baseline range, and analyzing the heart rate sequence data in the computational model component to determine the probability.

Document EP 3 591 659 A1 discloses embodiments that are directed to systems and methods for managing hot flashes and/or menopause symptoms. An example system includes sensor circuitry and logic circuitry. The sensor circuitry obtains a physical measurement associated with a user and communicates the physical measurement. The logic circuitry generates a predictive model that indicates a probability of the user having a hot flash at a date and time based on a plurality of input parameters, revises the probability based on the physical measurement using the predictive model, and communicates data indicative of an action in response to the revised probability being outside a threshold, such as providing cooling relief.

It can be seen as an object of the invention to provide a computer implemented method and a computer implemented system that solve the problems in the prior art, and in particular to provide a computer implemented method and a computer implemented system for efficiently, securely and reliably determining a menopausal state.

The object is solved by the subject-matter of the independent claims. Advantageous further developments are subject-matter of the dependent claims. Disclosed is a computer implemented method according to independent claim 1.

Advantageously, in step c), the measured body condition information is a time series of measured body conditions, respectively.

Advantageously, in step d) the computer implemented classification model is a decision tree model or a random forest model or an artificial neural network model, in particular a recurrent neural network model or a convolutional neural network model, further in particular an echo state network model or a liquid state machine model.

Advantageously, in step a), the set of body condition information contains a subset of the set: electrodermal activity, heart rate, in particular photoplethysmographic information and electrocardiogram information, body temperature, body movement activity, in particular relocation or acceleration, body environment information, in particular ambient temperature, ambient humidity or ambient pressure, Bioimpedance, EMG, EEG, Ultrasound, NIRS, Microbiota (vaginal or gut), pH, Voice patterns, Breathing pattern and frequency, Interstitial fluid deviations.

Advantageously, step c) further comprises the step: ca) measuring electrodermal activity and providing measured electrodermal activity information, and step d) further comprises the steps: da) transforming the measured electrodermal activity information into SCL information and SCR information, and db) providing the SCL information or the SCR Information as measured body condition information, and step f) comprises the step: fa) inputting the SCL information or the SCR information as measured body condition information to the classification model.

Advantageously, step h) further comprises the step: ha) providing the SCL information with the highest weight of the set of measurable body conditions for classification.

Advantageously, step d) further comprises at least one of the steps: dc) applying a low pass filter, in particular a Butterworth filter, in particular a Butterworth filter with a cut off frequency of 0.5 Hz, to the measured body condition information, dd) adapting a sampling rate of the measured body condition information to 1 Hz for the measurable body conditions of the set of measurable body conditions, de) smoothen the measured body condition information by applying a sliding left windows of 60 seconds, in particular by determining a simple moving average by forming the unweighted mean of the previous 60 measured samples, df) providing the measured body condition information with classification information such that the measured body condition information that occur within a time period of 180 seconds preceding and of 180 seconds succeeding a point in time of the detection of a symptom of a menopausal state are labeled as belonging to the symptom of the menopausal state, and that the measured body condition information that occur outside that time period are labeled as not belonging to a symptom of a menopausal state.

Advantageously, step d) further comprises step: dg) applying an additional filter to the measured body condition information based on measured body condition information.

Advantageously, in step dg) measured EDA information or measured T information is filtered if it occurs together with increased measured ambient temperature information or increased measured ACC information.

Advantageously, the classification model is located at a remote site, in particular, as a centralized cloud service.

Disclosed is a computer implemented method, not covered by the claims, for determining a menopausal state, comprising the steps: i) providing a classification model according to any of claims 1 to 10, j) determining a diagnose object, the diagnose object being a human being capable of adopting a menopausal state and having an unknown state concerning their menopausal state as menopausal state information, k) arranging at least one sensor at the body of the diagnose object at a sensor body location, l) measuring, by means of the at least one sensor, a set of measurable body conditions from a group of body conditions of a human body, the body conditions being indicative of a menopausal state of a human body and being a subset of the set of measurable body conditions according to step a), as measured diagnose body condition information, m) preprocessing the measured diagnose body condition information to provide preprocessed diagnose body condition information, n) inputting the preprocessed diagnose body condition information of the diagnose object as diagnose input information to the classification model, o) classifying a menopausal state of the diagnose object as menopausal state information, p) using the classified menopausal state information for providing a diagnose on the menopausal state of the diagnose object.

Advantageously, in step l), the measured diagnose body condition information is a time series of measured body conditions, respectively.

Advantageously, in step i) the computer implemented classification model is a decision tree model or a random forest model or an artificial neural network model, in particular a recurrent neural network model or a convolutional neural network model, further in particular an echo state network model or a liquid state machine model.

Advantageously, in step l), the set of body condition information contains a subset of the set: electrodermal activity, heart rate, in particular photoplethysmographic and electrocardiogram (ECG) information, body temperature, body movement activity, in particular relocation or acceleration, body environment information, in particular ambient temperature, ambient humidity or ambient pressure, Bioimpedance, EMG, EEG, Ultrasound, NIRS, Microbiota (vaginal or gut), pH, Voice patterns, Breathing pattern and frequency, Interstitial fluid deviations.

Advantageously, step I) further comprises the step: la) measuring electrodermal activity and providing measured diagnose electrodermal activity information, and step m) further comprises the steps: ma) transforming the measured diagnose electrodermal activity information into SCL information and SCR information, and mb) providing the SCL information or the SCR Information as measured diagnose body condition information, and step n) comprises the step: na) inputting the SCL information or the SCR information as measured diagnose body condition information to the classification model.

Advantageously, step o) further comprises the step: oa) considering the SCL information with the highest weight of the set of measurable body conditions for classification.

Advantageously, step m) further comprises at least one of the steps: mc) applying a low pass filter, in particular a Butterworth filter, in particular a Butterworth filter with a cut-off frequency of 0.5 Hz, to the measured diagnose body condition information, md) adapting a sampling rate of the measured diagnose body condition information to 1 Hz for the measurable body conditions of the set of measurable body conditions, me) smoothen the measured diagnose body condition information by applying a sliding left windows of 60 seconds, in particular by determining a simple moving average by forming the unweighted mean of the previous 60 measured samples, or mf) applying an additional filter to the measured diagnose body condition information based on measured diagnose body condition information.

Advantageously, the method further comprises the step: dg) providing a sensor unit at a sensor location, wherein the sensor location is arranged at a human body, in particular at a torso, in particular at the lower thorax, in particular below the sternum, either centrally or at the frontal left side of the thorax of the diagnose object.

Disclosed is a computer implemented system to classify a menopausal state, according to independent claim 8.

Advantageously, the sensor unit, the transmission unit or the evaluation unit is configured to perform step m).

Advantageously, the system is configured to perform any of the previous computer implemented methods for determining a menopausal state.

Advantageously, the sensor unit further comprises a sensing portion at a proximal side of the sensor unit, the sensing portion comprising a convex shape, and an urging portion that is configured to urge the sensing portion towards a dermal surface at a sensor location, such that the sensing portion indents the dermal surface.

Advantageously, the/a sensor location is arranged at a human body, in particular at a torso, in particular at the lower thorax, in particular below the sternum, either centrally or at the frontal left side of the thorax.

Disclosed is a computer implemented method for establishing a weighted set of measurable body conditions, comprising the steps: p) providing a classification model according to any of claims 1 to 10, q) determining a set of control objects, the control objects being humans being capable of adopting a menopausal state and having a known state concerning their menopausal state as menopausal state information, r) arranging at least one sensor at the body of the control object at a sensor body location, s) measuring, by means of the at least one sensor, a set of measurable body conditions from a group of body conditions of a human body, the body conditions being indicative of a menopausal state of a human body and being a subset of the set of measurable body conditions according to step a), as measured control body condition information, t) preprocessing the measured control body condition information to provide preprocessed control body condition information, u) inputting the preprocessed control body condition information of the control object as control input information to the classification model, v) classifying a menopausal state of the control object as classified menopausal state information, w) comparing the classified menopausal state information with the menopausal state information of the control object, x) determining a significance of a member of the set of measurable body conditions for classification of the menopausal state for all members of the set of measurable body conditions, y) providing a weight of the measured body condition information corresponding to the significance of the measurable body condition and adapting the classification model according to the weights of the measured body conditions.

Advantageously, the method further comprises the step z) reducing the set of measurable body conditions by removing an at least one least significant member.

Advantageously, step s) further comprises the step: sa) measuring electrodermal activity and providing measured electrodermal activity information, and step t) further comprises the steps: ta) transforming the measured electrodermal activity information into SCL information and SCR information, and tb) providing the SCL information or the SCR Information as measured body condition information, and step u) comprises the step: ua) inputting the SCL information or the SCR information as measured body condition information to the classification model.

Advantageously, step y) further comprises the step: ya) providing the SCL information with the highest weight of the set of measurable body conditions for classification.

Disclosed is a computer readable computer program product storing computer executable instructions that, when executed by the computer system, makes the computer system performing any one of the above methods.

Disclosed is a computer implemented system for understanding menopausal progression that is configured to use a sensor circuitry in combination with a logic circuitry, the logic circuitry implementing a predictive model that indicates the time to menopause onset.

Disclosed is a computer implemented system that is configured to use continuous biometric measurements for perimenopausal, menopausal and post-menopausal diagnose objects to derive predictions and recommendations for treatment and medication of menopausal symptoms.

### BRIEF DESCRIPTION OF THE DRAWINGS

In the following, the invention is explained by means of an embodiment and the figures.
**Figure 1** exhibits a schematic view of an embodiment of a computer implemented system for determining a menopausal state.
**Figure 2** exhibits a detailed view of a sensor unit according to the embodiment.
**Figure 3** exhibits a flow chart of an embodiment of a computer implemented method for providing a model for determining a menopausal state.
**Figure 4** exhibits a flow chart of a preprocessing process of the computer implemented method for providing a model for determining a menopausal state.
**Figure 5** exhibits a flow chart of a training process of the computer implemented method for providing a model for determining a menopausal state.
**Figure 6** exhibits a flow chart of an embodiment of a computer implemented method for determining a menopausal state.
**Figure 7** exhibits a flow chart of a preprocessing process of the computer implemented method for determining a menopausal state.
**Figure 8** exhibits a schematic view of positioning of a sensor.

### DETAILED DESCRIPTION OF THE DRAWINGS

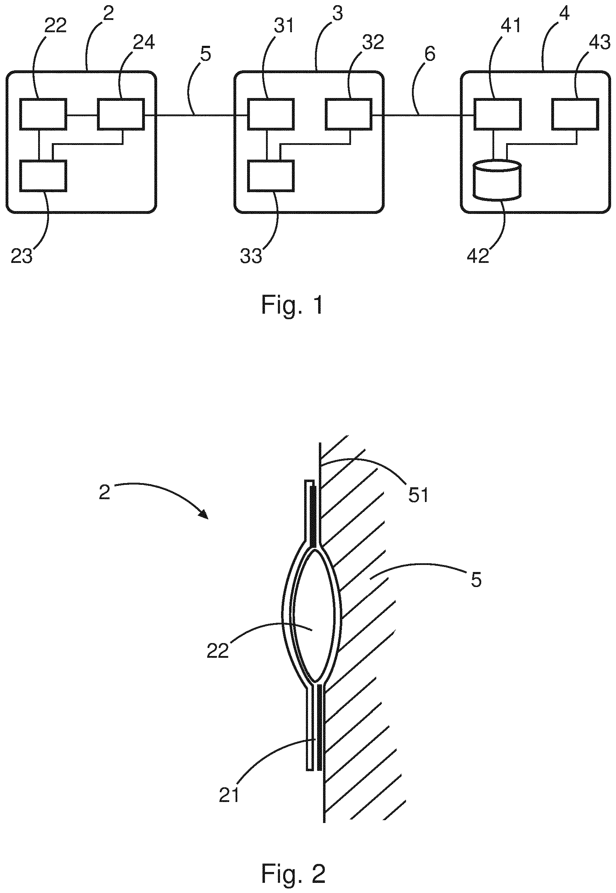

By means of **Fig. 1** and **Fig.2****,** an embodiment of a computer implemented system 1 for determining a menopausal state is described.

The system 1 comprises a sensor unit 2, a transmission unit 3 and an evaluation unit 4.

The sensor unit 2 is a supra dermal wearable sensor that is fixed to a sensor location 52 on a dermal surface 51 of a human body 5 by means of a mechanical fixing method. In the current embodiment, all relevant information may be collected by a supra dermal wearable sensor. In further developments, however, data collected by a intradermal sensor, in particular a needle penetrating the skin for measurements of body fluids may be used. In the embodiment, the fixing method is adhesion by an adhesive layer 21. Alternatively, positive locking, e.g. by means of an elastic belt may be employed.

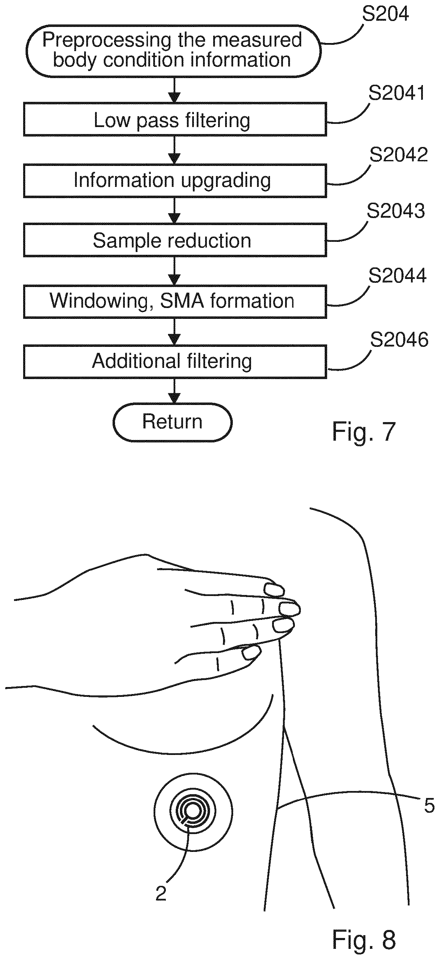

As shown in **Fig. 8**, I sensor location 52 in the embodiment is at the torso, preferably at the lower thorax, preferably below the sternum, either centrally or at the frontal left side of the thorax. This sensor location 52 exhibited better sensor signal quality than other sensor location of wearable sensors known from the related art like wrists.

The sensor unit 2 comprises a sensing portion 22 that is capable of detecting photoplethysmographic (PPG) information, electrocardiogram (ECG) information, skin temperature (T) information acceleration/motion (ACC) information and electrodermal activity (EDA) information. PPG information is detected at a sample rate of 50-100 Hz. ECG information is detected at a sample rate of 100-500 Hz. EDA information is detected at a sample rate of 4-30 Hz. T information is detected at a sample rate of 0.2-1 Hz. ACC information is detected at a sample rate of 25-100 Hz. The sensing portion 21 is located at the proximal side of the sensor and comprises a convex projection. Due to the convex shape, the sensing portion 22 is firmly urged towards the skin and thus provides a secure contact area between the skin surface and the sensing portion 22, thus improving the detection of sensor information, in particular improving PPG sensor information.

The sensor unit 2 comprises a sensor communication portion 24 that is configured to establish a wireless short range communication link 6 with the transmission unit 3, e.g. via a transmission protocol according to IEEE 802.15.1 (Bluetooth). Other wireless transmission protocols like protocols according to IEEE 802.11 or proprietary protocols may be used. Raw sensor data is transmitted via the short range communication link 6.

The sensor unit 2 comprises a sensor stored energy source 24, e.g. a rechargeable or non-rechargeable battery, that supplies the sensing portion 21 and the sensor communication portion 24 with electric energy for operation.

The sensor stored energy source 24 is removable from the sensor unit 2 and replaceable to extend the device's battery life when changed. Allowing the user to swap out the battery without removing the sensor unit 2 from the human body 5. The sensor stored energy source 24, when being a rechargeable battery, is recharged in a charging case (not shown) designed specifically to hold the sensor stored energy source 24, the charging case is connected to an electrical outlet through a cable.

The transmission unit 3 is a transceiver that establishes the short-range transmission link 6 with the sensor communication portion 24 via a short-range communication portion 31. The transmission unit 3 preferably is a mobile device, preferably a smart phone. The transmission unit 3 comprises a long-range communication unit 32, e.g. a Wi-Fi module or a cellphone module to establish a wireless long range communication link 7 either directly or indirectly, e.g. via the internet, with the evaluation unit 4, e.g. via a transmission protocol according to IEEE 802.11 or according to GSM, UMTS, LTE or the like. Raw sensor data is transmitted via the long-range communication link 7 to the evaluation unit 4.

The evaluation unit 4 is an electronic data processing device comprising a data storage 42 and implementing a classification model 43. In this embodiment, the classification model 43 is a random forest model. Alternatively, other classification models, in particular decision trees or deep learning methods and artificial neural networks may be employed. The evaluation unit comprises a network interface 42 that is configured to establish the long-range transmission link 7 with the transmission unit 3 via the long-range communication portion 32.

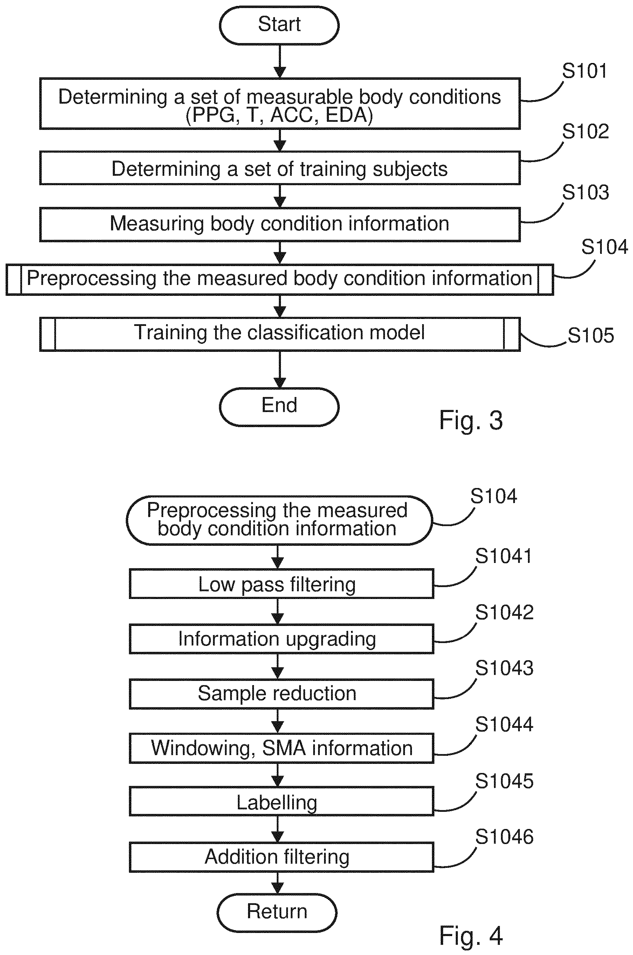

By means of **Fig. 3, Fig. 4** **and** **Fig. 5****,** an embodiment of a computer implemented method for establishing a classification model for determining a menopausal state is described.

**Fig. 3** shows a flow chart of an embodiment of a method for establishing a classification model for determining a menopausal state.

In a parameter determining step S101, a set of measurable body conditions from a group of body conditions of a human body are determined as measured parameters that may be indicative of a menopausal state of a human body. In the embodiment, the menopausal state that is intended to be determined is perimenopause. In the embodiment, the set of measurable body conditions corresponds to the available sensor information measured by the sensor unit 2 and output as measured body condition information. In the embodiment, the set of measurable body conditions comprise photoplethysmographic (PPG) properties, electrocardiogram (ECG properties), skin temperature (T) properties, acceleration/motion (ACC) properties and electrodermal activity (EDA) properties.

In a training object determining step S102, a number, that is sufficiently large according to known statistic principles, of human participants is selected as training objects, whereas the menopausal state of the participants is known. I.e. it is known whether a training object is in a perimenopausal state or not.

In a data acquisition step S103, the training objects are provided with sensors, in particular a sensor like the sensor unit 2, to measure and record the set of measurable body conditions, i.e. to measure and record photoplethysmographic (PPG) properties, electrocardiogram (ECG) properties, skin temperature (T) properties, acceleration/motion (ACC) properties and electrodermal activity (EDA) properties of the training object. The data acquisition step provides measured body condition information containing raw PPG data, raw ECG data, raw T data, raw ACC data and raw EDA data. In the embodiment, raw PPG data is a time series of PPG information recorded at a sampling rate of 50-100 Hz, ECG information is recorded at a sample rate of 100-500 Hz, raw EDA data is a time series of EDA information recorded at a sampling rate of 4-30Hz, raw T data is a time series of temperature information recorded at a sampling rate of 0.2-1 Hz and raw ACC data is a time series of acceleration information recorded at a sampling rate of 25-100 Hz. At the same time, it is recorded, whether the measured body condition information is associated with a perimenopausal state or with a symptom of a perimenopausal state, either deriving from the affiliation of the training object to one of the two groups of experiencing/not experiencing a perimenopausal state or indication that the training object currently experiences/does not experience perimenopausal symptoms.

In a data preprocessing step S104, the measured body condition information is preprocessed to gain preprocessed body condition information. The data preprocessing step S104 is described in more detail by referring to **Fig. 4****.**

In a filtering sub step S1041, the measured body condition information is subjected to a low pass filter to reduce signal noise. In the embodiment, the low pass filter is a Butterworth low pass filter with a cut off frequency of 0.5 Hz.

In an upgrading sub step S1042, the measured or hitherto preprocessed body condition information is aggregated to higher level information. The raw PPG data is converted to a time series of heart rate information, heart rate variability and blood pressure (with time). The raw T data is converted to a time series of body temperature information. The raw ACC data is converted to a time series of activity information, i.e. information that indicate a level of body activity over time. The raw EDA data is converted to time series of electrodermal phasic sympathetic activity (SCR) information and electrodermal tonic sympathetic activity (SCL) information.

In a sample reduction sub step S1043, the sampling rate of the measured or hitherto preprocessed body condition information is synchronized and reduced to 1 Hz. This considerably reduces the amount of data intended to be processed.

In a windowing sub step S1044, the time series of hitherto preprocessed body condition information is smoothened by determining a simple moving average (SMA) by forming the unweighted mean of the previous 60 data points, i.e., the unweighted mean of the values of preceding 60 samples of the measurements of each parameter.

In a labelling sub step S1045, the measured or hitherto preprocessed body condition information of 180 seconds respectively preceding or succeeding the detection of occurrence of a perimenopausal symptoms are labelled as belonging to a perimenopausal symptom as classification information. I.e. the point in time at that the training object detects and indicates occurrence of a perimenopausal symptom is recorded. Those measured or hitherto preprocessed body condition information, i.e. any sample thereof occurring in a time span of 180 seconds respectively preceding or succeeding that point in time is marked as belonging to a perimenopausal symptom, e.g. by setting a labelling bit to 1, while samples of measured or preprocessed body condition information outside of this 360 second time period are marked as not belonging to a perimenopausal symptom, e.g. by setting the labelling bit to 0. Thus, samples of measured or hitherto preprocessed body condition information are provided with classification information indicating whether the respective sample belongs to a symptom or not.

In an optional filtering sub step S1046, additional filtering of the measured or hitherto preprocessed body condition information or preprocessed body condition information may be applied in accordance with the measured body condition information or preprocessed body condition information. Thus, a peak in the time series of body temperature information may be dampened if it occurs together with a peak in the time series of activity information, in order to eliminate body temperature variations that follow from physical activity.

Additionally, or alternatively, a peak in the time series of body temperature information may be dampened if it occurs together with a rise of the ambient temperature if corresponding information is available, in order to eliminate body temperature variations that follow from ambient temperature variations.

Additionally, or alternatively, a peak in the time series of SCR information or SCL information may be dampened that occurs together with a rise in ambient temperature or a peak in activity information, in order to eliminate EDA variations that follow from increased perspiration following from physical activity or increased ambient temperature.

By the preprocessing step S104, the amount of data intended to be processed is significantly reduced in comparison to the related art approaches. Nevertheless, due to the specific data preprocessing, reliable classification results are still achieved. In particular in conjunction with the sensor unit 2 and the sensor location 52, reliable classification results are still achieved.

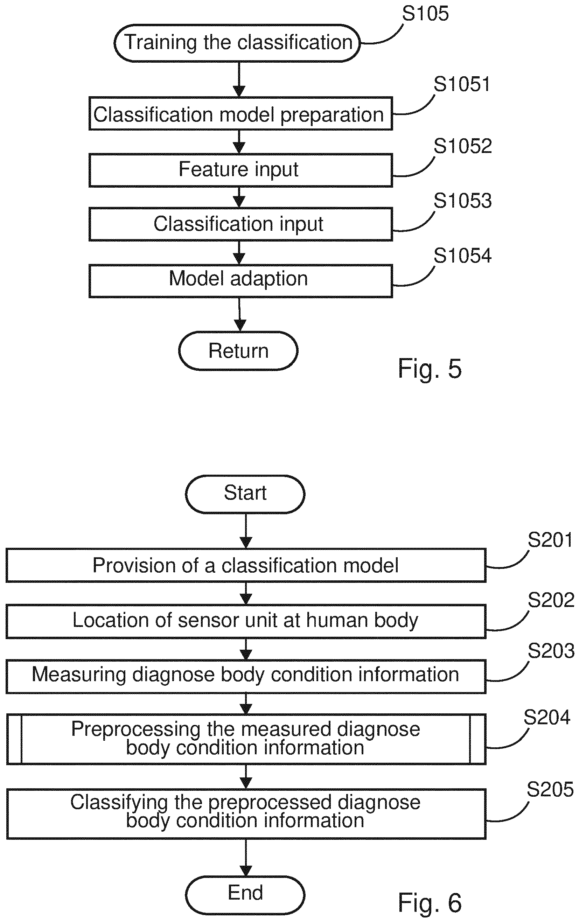

In a training step S105, a computer implemented classification model is generated. The training step S104 is described in more detail by referring to **Fig. 5****.**

In a preparation sub step S1051, a computer implemented self-learning classification model is established. In the current embodiment, the self-learning classification model is a random forest model. In this random forest model, a set of decision trees is modelled such that a subset of features of the available preprocessed information is selected at random and assigned to the tree nodes.

In a feature input step S1052, the preprocessed body condition information consisting of a sample of a set of preprocessed body condition information of labelling sub step S1045 is input to the random forest model as training input information.

In a classification input step S1053, the corresponding classification information assigned to the training input information is input to the random forest model as training classification information. I.e., it is input whether the set of preprocessed body condition information of feature input step S1052 belongs to measured body condition information that was measured during occurrence of a menopausal state.

In a model adaption step S1054, the random forest model is adapted to the training input information and training classification information.

Feature input step S1052, classification input step S1053 and model adaption step S1054 are performed for all preprocessed body condition information.

As a result, there is a trained classification model to classify a perimenopausal state or the absence thereof. The thus gained classification model is used to diagnose a perimenopausal state of diagnose objects.

By means of **Fig. 6****,** an embodiment of a computer implemented method for determining a menopausal state of a diagnose object is described.

In a classification model provision step S201, a classification model is trained according to the previously described method according to the method steps S101 through S105.

In a sensor positioning step S202, a sensor unit 2 is positioned at the sensor location 52 at a body of the diagnose object and is activated. The sensor location is positioned at the torso, particularly at the lower thorax, particularly below the sternum, either centrally or at the frontal left side of the thorax.

In a diagnose data acquisition step S203, the set of measurable body conditions are measured and recorded, i.e., photoplethysmographic (PPG) properties, electrocardiogram (ECG) properties, skin temperature (T) properties, acceleration/motion (ACC) properties and electrodermal activity (EDA) properties of the diagnose object. The diagnose data acquisition step provides measured diagnose body condition information containing raw PPG data, raw ECG data, raw T data, raw ACC data and raw EDA data. In the embodiment, raw PPG data is a time series of PPG information recorded at a sampling rate of 50-100 Hz, raw ECG information is is a time series of recorded at a sample rate of 100-500 Hz, raw EDA data is a time series of EDA information recorded at a sampling rate of 4-30 Hz, raw T data is a time series of temperature information recorded at a sampling rate of 0.2-1 Hz and raw ACC data is a time series of acceleration information recorded at a sampling rate of 25-100 Hz.

In a diagnose data preprocessing step S204, the measured body condition information is preprocessed to gain preprocessed diagnose body condition information. The diagnose data preprocessing step S204 is similar to the data preprocessing step S104 as described above by referring to **Fig. 4** with some deviations as described by referring to **Fig. 7****.**

In a filtering sub step S2041, the measured body condition information is subjected to a low pass filter to reduce signal noise. In the embodiment, the low pass filter is a Butterworth low pass filter with a cut off frequency of 0.5 Hz.

In an upgrading sub step S2042, the measured or hitherto preprocessed body condition information is aggregated to higher level information. The raw PPG data is converted to a time series of heart rate information, heart rate variability and blood pressure (with time). The raw T data is converted to a time series of body temperature information. The raw ACC data is converted to a time series of activity information, i.e. information that indicate a level of body activity over time. The raw EDA data is converted to time series of electrodermal phasic sympathetic activity (SCR) information and electrodermal tonic sympathetic activity (SCL) information.

In a sample reduction sub step S2043, the sampling rate of the measured or hitherto preprocessed body condition information is synchronized and reduced to 1 Hz. This considerably reduces the amount of data intended to be processed.

In a windowing sub step S2044, the time series of hitherto preprocessed body condition information is smoothened by determining a simple moving average (SMA) by forming the unweighted mean of the previous 60 data points, i.e., the unweighted mean of the values of preceding 60 samples of the measurements of each parameter.

In an optional filtering sub step S1046, additional filtering of the measured or hitherto preprocessed body condition information or preprocessed body condition information may be applied in accordance with the measured body condition information or preprocessed body condition information. Thus, a peak in the time series of body temperature information may be dampened if it occurs together with a peak in the time series of activity information, in order to eliminate body temperature variations that follow from physical activity.

Additionally, or alternatively, a peak in the time series of body temperature information may be dampened if it occurs together with a rise of the ambient temperature if corresponding information is available, in order to eliminate body temperature variations that follow from ambient temperature variations.

Additionally, or alternatively, a peak in the time series of SCR information or SCL information may be dampened that occurs together with a rise in ambient temperature or a peak in activity information, in order to eliminate EDA variations that follow from increased perspiration following from physical activity or increased ambient temperature.

By the preprocessing step S104, the amount of data intended to be processed is significantly reduced in comparison to the related art approaches. Nevertheless, due to the specific data preprocessing, reliable classification results are still achieved. In particular in conjunction with the sensor unit 2 and the sensor location 52, reliable classification results are still achieved.

In a diagnose data classifying step S205, samples of sets of the preprocessed diagnose body condition information are input into the classification model and the perimenopausal state of the diagnose object is classified by the classification model as diagnose classification information. For that, in the embodiment, the preprocessed diagnose body condition information is input into the random forest model and each decision tree of the random forest model is processed. Each decision tree achieves a local classification classifying the diagnose object either being in a perimenopausal state or not being in a perimenopausal state. The classification result of the random forest model is determined to be the one classification that is adopted by the majority of the decision trees.

The diagnose classification is recorded, output to the test object, e.g., via the transmission unit 3 or to an attending physician.

It occurs that in contrast to comparable fields like anxiety detection, where electrodermal phasic sympathetic activity is a significant marker, in detection of a perimenopausal state, surprisingly electrodermal tonic sympathetic activity is the more significant marker.

The invention was described by means of an embodiment.

As recognizable by the skilled person, deviations from the embodiment are possible without leaving the invention that is defined according to the scope of the claimed object-matter.

For example, in the embodiment, a random forest model was used as a classification model. Random forest models provide reliable classification results with moderate computational resource requirements. Alternatively, a decision tree may be employed as a classification model, requiring less computational resources. Alternative, deep learning methods and an artificial neuronal network may be employed as a classification model, providing an improved scalability.

In the embodiment, raw sensor data recorded by the sensor unit 2 is transmitted to the evaluation unit 4 via the transmission unit 3 and is pre-processed at the evaluation unit 4. Alternatively, the raw sensor data may be pre-processed by the sensor unit 2 or the transmission unit 3 to reduce the required data traffic.

In the embodiment, the menopausal state of perimenopause was classified. Alternatively, with corresponding training data, premenopause or postmenopause may be classified.

In this document, the terms "and", "or" and "either ... or" are used as conjunctions in a meaning similar to the logical conjunctions "AND", "OR" (often also "and/or") or "XOR", respectively. In particular, in contrast to "either ... or", the term "or" also includes occurrence of both operands ("and/or").

Method steps indicated in the description or the claims only serve an enumerative purpose of the method steps. They only imply a given sequence or an order where their sequence or order is explicitly expressed or is - obvious for the skilled person - mandatory due to their nature. In particular, the listing of method steps do not imply that this listing is exhaustive. Further steps may be interposed. Also, not all method steps described in an embodiment are required to implement the invention. The required method steps are defined by the claims.

In the description and claims, the term "comprising" does not exclude existence or presence of other elements or steps. The indefinite article "a" or "an" does not exclude a plurality and has to be understood as "at least one".

Further, in this document, the following terms are understood in the following given meaning.

**Menopausal state** is the condition of a human body concerning the age-related loss of capability of ovarian generation of fertile ova through ovulation, usually recognized by the last occurrence of a menstruation. In the scope of this document, three menopausal states are distinguished: premenopause, perimenopause and postmenopause. Different menopausal states may exhibit different symptoms, respectively, and provide different options for mitigation of possible disorders, respectively.

**Premenopause** denotes a time period consisting of years leading up to the last menstruation, when the levels of reproductive hormones are becoming more variable and lower, and the effects of hormone withdrawal are present. Premenopause starts some time before the menstrual cycles become noticeably irregular in timing.

**Perimenopause** denotes a time period of the menopausal transition years before the date of the final menstruation. This transition may, dependent on the source, last for about four to eight years or six- to ten-years, ending 12 months after the last menstruation.

**Postmenopause** denotes a time period starting 12 months after the final menstruation, also indicated by a considerably increased FSH level of 16.74-113.59 mlU/ml.

**Measurable body condition** is a property of a human body that is accessible to a measurement by a sensor. Examples for measurable body conditions are skin temperature measurable by a thermometer, skin conductance/electrodermal activity like EDR and EDL, skin reflectance/skin light absorbance measurable by PPG, electric current flow through the heart measurable by ECG, tissue layer thicknesses measurable by ultrasonic sensors, liquid flow rates like blood flow rates measurable by ultrasonic sensors, acoustic noise emission spectrum as measurable by acoustic sensors or stethoscopes, body movement activity measurable by accelerometers attached to a human body, or the like.

**Measured body condition information** is data that a sensor directly or indirectly derives and outputs when measuring a measurable body condition. This data may be directly measured information, e.g. a value of light reflectance/absorbance of human skin, or deduced information like blood volume changes or blood oxygen saturation derived from changes of light reflectance/absorbance of human skin.

**Skin conductance (SC)/ electrodermal activity (EDA)** is the property of the human body that causes continuous variation in the electrical characteristics of the skin. EDA may be measured electrically, alternatively or cumulatively by skin potential, skin resistance, skin conductance, skin admittance and skin impedance.

**Electrodermal phasic sympathetic activity (EDR, SCR)** are short lasting changes in EDA giving an objective, transient indication of autonomic nervous system arousal in response to a stimulus.

**Electrodermal tonic sympathetic activity (EDL, SCL)** are long lasting changes in EDA. They are generally considered to be the level of skin conductance in the absence of any particular discrete environmental event or external stimuli.

**Photoplethysmography (PPG)** is the optically obtaining of a plethysmogram that can be used to detect blood volume changes in the microvascular bed of tissue by measuring the property of the human body's skin to absorb/reflect light.

**Electrocardiogram (ECG)** is a measure of the electrical current that spreads through the heart during a cardiac impulse. A small amount of this current spreads to the skin and the electrical potential generated by the current can be detected by electrodes placed on the skin at opposite sides of the heart.

**Classification** describes the allocation of an object, e.g., an examined human, to a group of objects having one or more predetermined properties, e.g. to humans having a specific menopausal state.

**Decision tree models** are a known supervised learning approach used in statistics, data mining and machine learning. In this formalism, a classification or regression decision tree is used as a predictive model to draw conclusions about a set of observations. Tree models where the target variable can take a discrete set of values are called classification trees; in these tree structures, leaves represent class labels and branches represent conjunctions of features that lead to those class labels. Decision trees where the target variable can take continuous values (typically real numbers) are called regression trees. More generally, the concept of regression tree can be extended to any kind of object equipped with pairwise dissimilarities such as categorical sequences.

**Random forest models** are a known ensemble learning method for classification, regression and other tasks that operate by constructing a multitude of decision trees at training time. For classification tasks, the output of the random forest is the class selected by most trees. For regression tasks, the mean or average prediction of the individual trees is returned.

**Deep learning models** are a known family of machine learning methods based on artificial neural networks with representation learning. Learning can be supervised, semi-supervised or unsupervised. Deep learning models are a class of machine learning algorithms that use multiple layers to progressively extract higher-level features from a raw input.

### LIST OF REFERENCE SIGNS

- 1: computer implemented system for determining a menopausal state (first embodiment)
- 2: sensor unit
- 21: adhesive layer
- 22: sensing portion
- 23: sensor stored energy source, battery
- 24: sensor communication portion
- 3: transmission unit, mobile device, smart phone
- 31: short range communication portion
- 32: long range communication portion, Wi-Fi module, cellphone module
- 33: cellphone energy source, battery
- 4: evaluation unit
- 41: network interface
- 42: data storage
- 43: classification model
- 5: human body
- 51: dermal surface
- 52: sensor location
- 6: short range transmission link
- 7: long range transmission link, GSM network, internet

## Claims

1. A computer implemented method for providing a classification model to classify a menopausal state, the method being performed by an evaluation unit of a system, the evaluation unit comprising a data storage (42) and implementing a classification model (43) and receiving raw sensor data via a long-range communication link (7), the method comprising the steps:
a) determining a set of measurable body conditions from a group of body conditions of a human body, the body conditions being indicative of a menopausal state of a human body,
b) determining a set of training objects, the training objects being humans being capable of adopting a menopausal state and having a known state concerning their menopausal state as menopausal state information,
c) measuring, by sensors that the training objects are provided with, the measurable body conditions of the set of measurable body conditions of each of the training objects for a predetermined amount of time to provide measured body condition information for each of the training objects, and detecting occurrence of a symptom of a menopausal state,
d) preprocessing the measured body condition information to provide preprocessed body condition information, by applying a low pass filter, reducing a sampling rate, applying a sliding left window or labelling perimenopausal symptoms as classification information,
e) providing the computer implemented classification model adapted to classify a menopausal state, i.e. a trained classification model to classify a perimenopausal state or the absence thereof,
f) inputting the preprocessed body condition information of a training object as training input information to the classification model,
g) inputting the detected occurrence of the symptom of a menopausal state as training classification information to the classification model,
h) adapting the classification model according to the training input information and training classification information.

2. The method according to claim 1, wherein
in step c), the measured body condition information is a time series of measured body conditions, respectively.

3. The method according to any of claims 1 to 2, wherein
in step e) the computer implemented classification model is a decision tree model or a random forest model or an artificial neural network model, in particular a recurrent neural network model or a convolutional neural network model, further in particular an echo state network model or a liquid state machine model.

4. The method according to any one of claims 1 to 3, wherein
in step a), the set of body condition information contains a subset of the set:
- electrodermal activity,
- heart rate, HRV and Blood Pressure, in particular photoplethysmographic information and ECG information
- body temperature,
- body movement activity, in particular relocation or acceleration, or
- body environment information, in particular ambient temperature, ambient humidity or ambient pressure.

5. The method according to any of clams 1 to 4,
wherein step c) further comprises the step:
ca) measuring electrodermal activity and providing measured electrodermal activity information,
and step d) further comprises the steps:
da) transforming the measured electrodermal activity information into SCL information and SCR information, and
db) providing the SCL information or the SCR Information as measured body condition information,
and step f) comprises the step:
fa) inputting the SCL information or the SCR information as measured body condition information to the classification model.

6. The method according to any of clams 1 to 5,
wherein step h) further comprises the step:
ha) providing the SCL information with the highest weight of the set of measurable body conditions for classification.

7. The method according to any of claims 1 to 6,
wherein step d) further comprises at least one of the steps:
dc) applying a low pass filter, in particular a Butterworth filter, in particular a Butterworth filter with a cut off frequency of 0.5 Hz, to the measured body condition information,
dd) adapting a sampling rate of the measured body condition information to 1 Hz for the measurable body conditions of the set of measurable body conditions,
de) smoothen the measured body condition information by applying a sliding left windows of 60 seconds, in particular by determining a simple moving average by forming the unweighted mean of the previous 60 measured samples,
df) providing the measured body condition information with classification information such that the measured body condition information that occur within a time period of 180 seconds preceding and of 180 seconds succeeding a point in time of the detection of a symptom of a menopausal state are labeled as belonging to the symptom of the menopausal state, and that the measured body condition information that occur outside that time period are labeled as not belonging to a symptom of a menopausal state.

8. A computer implemented system (1) to classify a menopausal state, comprising:
a sensor unit (2),
a transmission unit (3), and
an evaluation unit (4), containing a classification model provided according to any of claims 1 to 7,
wherein the system is configured to perform the steps for a diagnose object, the diagnose object being a human being capable of adopting a menopausal state and having an unknown state concerning their menopausal state as menopausal state information, having at least one sensor at the body of the diagnose object at a sensor body location:
i) measuring, by means of the at least one sensor, a set of measurable body conditions from a group of body conditions of a human body, the body conditions being indicative of a menopausal state of a human body and being a subset of the set of measurable body conditions according to step a), as measured diagnose body condition information,
j) preprocessing - by the evaluation unit - the measured diagnose body condition information to provide preprocessed diagnose body condition information,
k) inputting - by the evaluation unit - the preprocessed diagnose body condition information of the diagnose object as diagnose input information to the classification model,
l) classifying a menopausal state of the diagnose object as menopausal state information,
m) using the classified menopausal state information for providing a diagnose on the menopausal state of the diagnose object, wherein
the sensor unit is configured to measure a set of measurable body conditions from a group of body conditions of a human body, the body conditions being indicative of a menopausal state of a human body and being a subset of the set of measurable body conditions and transmit measured body conditions as measured body condition information to the transmission unit (3),
the transmission unit (3) is configured to receive the body condition information from the sensor unit (2) and to transmit the body condition information to the evaluation unit (4), and
the sensor unit (2) is configured to perform the step i) and the evaluation unit (4) is configured to perform the steps k) and l).

9. The system according to claim 8, wherein
in step l), the set of body condition information contains a subset of the set:
- electrodermal activity,
- heart rate, heart rate variability and blood pressure in particular photoplethysmographic information and ECG information
- body temperature,
- body movement activity, in particular relocation or acceleration, or
- body environment information, in particular ambient temperature, ambient humidity or ambient pressure.

10. The system according to any of claims 8 to 9,
wherein step l) further comprises the step:
la) measuring electrodermal activity and providing measured diagnose electrodermal activity information,
and step m) further comprises the steps:
ma) transforming the measured diagnose electrodermal activity information into SCL information and SCR information, and
mb) providing the SCL information or the SCR Information as measured diagnose body condition information,
and step n) comprises the step:
na) inputting the SCL information or the SCR information as measured diagnose body condition information to the classification model,
or step o) further comprises the step:
oa) considering the SCL information with the highest weight of the set of measurable body conditions for classification.

11. The system according to any of claims 8 to 10,
wherein step m) further comprises at least one of the steps:
mc) applying a low pass filter, in particular a Butterworth filter, in particular a Butterworth filter with a cut-off frequency of 0.5 Hz, to the measured diagnose body condition information,
md) adapting a sampling rate of the measured diagnose body condition information to 1 Hz for the measurable body conditions of the set of measurable body conditions,
me) smoothen the measured diagnose body condition information by applying a sliding left window of 60 seconds, in particular by determining a simple moving average by forming the unweighted mean of the previous 60 measured samples,
mf) applying an additional filter to the measured diagnose body condition information based on measured diagnose body condition information, or
mg) providing a sensor unit at a sensor location (52), wherein the sensor location (52) is arranged at a human body, in particular at a torso, in particular at the lower thorax, in particular below the sternum, either centrally or at the frontal left side of the thorax of the diagnose object.

12. The system according to any of claims 8-11, wherein the sensor unit (2), the transmission unit (3) or the evaluation unit (4) is configured to perform step m).

13. The system according to any of the claims 8 to 12, wherein the sensor unit (2) further comprises
a sensing portion (22) at a proximal side of the sensor unit (2), the sensing portion comprising a convex shape , and
an urging portion (21) that is configured to urge the sensing portion towards a dermal surface (51) at a sensor location (52), such that the sensing portion indents the dermal surface.

## Patentansprüche

1. Computerimplementiertes Verfahren zum Bereitstellen eines Klassifizierungsmodells zur Klassifizierung eines menopausalen Zustands, wobei das Verfahren von einer Auswertungseinheit eines Systems durchgeführt wird und die Auswertungseinheit einen Datenspeicher (42) aufweist, ein Klassifizierungsmodell (43) implementiert und Sensor-Rohdaten über eine weitreichende Kommunikationsverbindung (7) empfängt, wobei das Verfahren folgende Schritte umfasst:
a) Bestimmen einer Menge von messbaren Körperzuständen aus einer Gruppe von Körperzuständen eines menschlichen Körpers, wobei die Körperzustände indikativ für einen menopausalen Zustand eines menschlichen Körpers sind,
b) Bestimmen einer Menge von Trainingssubjekten, wobei die Trainingssubjekte Menschen sind, die in der Lage sind, einen menopausalen Zustand anzunehmen, und einen bekannten Zustand bezüglich ihres menopausalen Zustands als menopausale Zustandsinformationen aufweisen,
c) Messen, durch Sensoren, mit denen die Trainingssubjekte versehen sind, der messbaren Körperzustände der Menge von messbaren Körperzuständen jedes der Trainingssubjekte für einen vorbestimmten Zeitraum, um gemessene Körperzustandsinformationen für jedes der Trainingssubjekte bereitzustellen, und Entdecken des Auftretens eines Symptoms eines menopausalen Zustands,
d) Vorverarbeiten der gemessenen Körperzustandsinformationen, um vorverarbeitete Körperzustandsinformationen bereitzustellen, durch Anwenden eines Tiefpassfilters, Reduzieren einer Abtastrate, Anwenden eines gleitenden linken Fensters oder Kennzeichnen von perimenopausalen Symptomen als Klassifizierungsinformationen,
e) Bereitstellen des computerimplementierten Klassifizierungsmodells, das dazu angepasst ist, einen menopausalen Zustand zu klassifizieren, d. h. eines trainierten Klassifizierungsmodells zur Klassifizierung eines perimenopausalen Zustands oder dessen Abwesenheit,
f) Eingeben der vorverarbeiteten Körperzustandsinformationen eines Trainingssubjekts als Trainingseingabeinformationen in das Klassifizierungsmodell,
g) Eingeben des entdeckten Auftretens des Symptoms eines menopausalen Zustands als Trainingsklassifizierungsinformationen in das Klassifizierungsmodell,
h) Anpassen des Klassifizierungsmodells anhand der Trainingseingabeinformationen und der Trainingsklassifizierungsinformationen.

2. Verfahren nach Anspruch 1, wobei
in Schritt c) die gemessenen Körperzustandsinformationen jeweils eine Zeitreihe von gemessenen Körperzuständen sind.

3. Verfahren nach einem der Ansprüche 1 bis 2, wobei
in Schritt e) das computerimplementierte Klassifizierungsmodell ein Entscheidungsbaum-Modell oder ein Random-Forest-Modell oder ein künstliches neuronales Netzwerkmodell ist, insbesondere ein rekurrentes neuronales Netzwerkmodell oder ein konvolutionelles neuronales Netzwerkmodell, ferner insbesondere ein Echo-State-Netzwerkmodell oder ein Liquid-State-Machine-Modell.

4. Verfahren nach einem der Ansprüche 1 bis 3, wobei
in Schritt a) die Menge von Körperzustandsinformationen eine Teilmenge folgender Menge enthält:
- elektrodermale Aktivität,
- Herzfrequenz, HRV und Blutdruck, insbesondere Photoplethysmographie-Informationen und EKG-Informationen,
- Körpertemperatur,
- Körperbewegungsaktivität, insbesondere Ortsänderung oder Beschleunigung, oder
- Körperumgebungsinformationen, insbesondere Umgebungstemperatur, Umgebungsluftfeuchte oder Umgebungsdruck.

5. Verfahren nach einem der Ansprüche 1 bis 4,
wobei Schritt c) ferner folgenden Schritt umfasst:
ca) Messen von elektrodermaler Aktivität und Bereitstellen von gemessenen elektrodermalen Aktivitätsinformationen;
Schritt d) ferner folgende Schritte umfasst:
da) Umwandeln der gemessenen elektrodermalen Aktivitätsinformationen in SCL-Informationen und SCR-Informationen und
db) Bereitstellen der SCL-Informationen oder der SCR-Informationen als gemessene Körperzustandsinformationen;
und Schritt f) folgenden Schritt umfasst:
fa) Eingeben der SCL-Informationen oder der SCR-Informationen als gemessene Körperzustandsinformationen in das Klassifizierungsmodell.

6. Verfahren nach einem der Ansprüche 1 bis 5,
wobei Schritt h) ferner folgenden Schritt umfasst:
ha) Bereitstellen der SCL-Informationen mit dem höchsten Gewicht der Menge von messbaren Körperzuständen für die Klassifizierung.

7. Verfahren nach einem der Ansprüche 1 bis 6,
wobei Schritt d) ferner mindestens einen der folgenden Schritte umfasst:
dc) Anwenden eines Tiefpassfilters, insbesondere eines Butterworth-Filters, insbesondere eines Butterworth-Filters mit einer Grenzfrequenz von 0,5 Hz, auf die gemessenen Körperzustandsinformationen,
dd) Einstellen einer Abtastrate der gemessenen Körperzustandsinformationen auf 1 Hz für die messbaren Körperzustände der Menge von messbaren Körperzuständen,
de) Glätten der gemessenen Körperzustandsinformationen durch Anwenden eines gleitenden linken Fensters von 60 Sekunden, insbesondere durch Bestimmen eines einfachen gleitenden Durchschnitts durch Bilden des ungewichteten Mittels der vorherigen 60 Messwerte,
df) Versehen der gemessenen Körperzustandsinformationen mit Klassifizierungsinformationen, sodass die gemessenen Körperzustandsinformationen, die innerhalb eines Zeitraums von 180 Sekunden vor und bis 180 Sekunden nach einem Zeitpunkt der Entdeckung eines Symptoms eines menopausalen Zustands auftreten, als zu dem Symptom des menopausalen Zustands gehörend gekennzeichnet werden und die gemessenen Körperzustandsinformationen, die außerhalb dieses Zeitraums auftreten, als nicht zu einem Symptom eines menopausalen Zustands gehörend gekennzeichnet werden.

8. Computerimplementiertes System (1) zum Klassifizieren eines menopausalen Zustands, aufweisend:
eine Sensoreinheit (2),
eine Übertragungseinheit (3) und
eine Auswertungseinheit (4), die ein bereitgestelltes Klassifizierungsmodell nach einem der Ansprüche 1 bis 7 enthält,
wobei das System dazu eingerichtet ist, folgende Schritte für ein Diagnosesubjekt durchzuführen, wobei das Diagnosesubjekt ein Mensch ist, der in der Lage ist, einen menopausalen Zustand anzunehmen, und einen unbekannten Zustand bezüglich seines menopausalen Zustands als menopausale Zustandsinformationen aufweist, und mindestens ein Sensor an dem Körper des Diagnosesubjekts an einer Körperposition vorhanden ist:
i) Messen, mittels des mindestens einen Sensors, einer Menge von messbaren Körperzuständen aus einer Gruppe von Körperzuständen eines menschlichen Körpers als gemessene Diagnose-Körperzustandsinformationen, wobei die Körperzustände indikativ für einen menopausalen Zustand eines menschlichen Körpers sind und eine Teilmenge der Menge von messbaren Körperzuständen gemäß Schritt a) sind,
j) Vorverarbeiten - durch die Auswertungseinheit - der gemessenen Diagnose-Körperzustandsinformationen, um vorverarbeitete Diagnose-Körperzustandsinformationen bereitzustellen,
k) Eingeben - durch die Auswertungseinheit - der vorverarbeiteten Diagnose-Körperzustandsinformationen des Diagnosesubjekts als Diagnose-Eingabeinformationen in das Klassifizierungsmodell,
I) Klassifizieren eines menopausalen Zustands des Diagnosesubjekts als menopausale Zustandsinformationen,
m) Verwenden der klassifizierten menopausalen Zustandsinformation zum Bereitstellen einer Diagnose des menopausalen Zustands des Diagnosesubjekts, wobei
die Sensoreinheit dazu eingerichtet ist, eine Menge von messbaren Körperzuständen aus einer Gruppe von Körperzuständen eines menschlichen Körpers zu messen, wobei die Körperzustände indikativ für einen menopausalen Zustand eines menschlichen Körpers sind und eine Teilmenge der Menge von messbaren Körperzuständen sind, und gemessene Körperzustände als gemessene Körperzustandsinformationen an die Übertragungseinheit (3) zu übertragen,
die Übertragungseinheit (3) dazu eingerichtet ist, die Körperzustandsinformationen von der Sensoreinheit (2) zu empfangen und die Körperzustandsinformationen an die Auswertungseinheit (4) zu übertragen, und
die Sensoreinheit (2) dazu eingerichtet ist, den Schritt i) durchzuführen, und die Auswertungseinheit (4) dazu eingerichtet ist, die Schritte k) und l) durchzuführen.

9. System nach Anspruch 8, wobei
in Schritt I) die Menge von Körperzustandsinformationen eine Teilmenge folgender Menge enthält:
- elektrodermale Aktivität,
- Herzfrequenz, Herzfrequenzvariabilität und Blutdruck, insbesondere Photoplethysmographie-Informationen und EKG-Informationen,
- Körpertemperatur,
- Körperbewegungsaktivität, insbesondere Ortsänderung oder Beschleunigung, oder
- Körperumgebungsinformationen, insbesondere Umgebungstemperatur, Umgebungsluftfeuchte oder Umgebungsdruck.

10. System nach einem der Ansprüche 8 bis 9,
wobei Schritt l) ferner folgenden Schritt umfasst:
la) Messen von elektrodermaler Aktivität und Bereitstellen von gemessenen Diagnoseinformationen über die elektrodermale Aktivität;
Schritt m) ferner folgende Schritte umfasst:
ma) Umwandeln der gemessenen Diagnoseinformationen über die elektrodermale Aktivität in SCL-Informationen und SCR-Informationen und
mb) Bereitstellen der SCL-Informationen oder der SCR-Informationen als gemessene Diagnose-Körperzustandsinformationen;
und Schritt n) folgenden Schritt umfasst:
na) Eingeben der SCL-Informationen oder der SCR-Informationen als gemessene Diagnose-Körperzustandsinformationen in das Klassifizierungsmodell;
oder Schritt o) ferner folgenden Schritt umfasst:
oa) Berücksichtigen der SCL-Informationen mit dem höchsten Gewicht der Menge von messbaren Körperzuständen für die Klassifizierung.

11. System nach einem der Ansprüche 8 bis 10, wobei Schritt m) ferner mindestens einen der folgenden Schritte umfasst:
mc) Anwenden eines Tiefpassfilters, insbesondere eines Butterworth-Filters, insbesondere eines Butterworth-Filters mit einer Grenzfrequenz von 0,5 Hz, auf die gemessenen Diagnose-Körperzustandsinformationen,
md) Einstellen einer Abtastrate der gemessenen Diagnose-Körperzustandsinformationen auf 1 Hz für die messbaren Körperzustände der Menge von messbaren Körperzuständen,
me) Glätten der gemessenen Diagnose-Körperzustandsinformationen durch Anwenden eines gleitenden linken Fensters von 60 Sekunden, insbesondere durch Bestimmen eines einfachen gleitenden Durchschnitts durch Bilden des ungewichteten Mittels der vorherigen 60 Messwerte,
mf) Anwenden eines zusätzlichen Filters auf die gemessenen Diagnose-Körperzustandsinformationen basierend auf gemessenen Diagnose-Körperzustandsinformationen oder
mg) Bereitstellen einer Sensoreinheit an einer Sensorposition (52), wobei die Sensorposition (52) an einem menschlichen Körper angeordnet ist, insbesondere an einem Torso, insbesondere am unteren Thorax, insbesondere unterhalb des Sternums, entweder zentral oder an der frontalen linken Seite des Thorax des Diagnosesubjekts.

12. System nach einem der Ansprüche 8-11, wobei die Sensoreinheit (2), die Übertragungseinheit (3) oder die Auswertungseinheit (4) dazu eingerichtet ist, Schritt m) durchzuführen.

13. System nach einem der Ansprüche 8 bis 12, wobei die Sensoreinheit (2) ferner aufweist:
einen Erfassungsabschnitt (22) an einer proximalen Seite der Sensoreinheit (2), wobei der Erfassungsabschnitt eine konvexe Form aufweist, und
einen drängenden Abschnitt (21), der dazu eingerichtet ist, den Erfassungsabschnitt in Richtung einer Hautoberfläche (51) an einer Sensorposition (52) zu drängen, sodass der Erfassungsabschnitt die Hautoberfläche eindrückt.

## Revendications

1. Procédé mis en œuvre par ordinateur permettant de fournir un modèle de classification pour classer un état de ménopause, le procédé étant exécuté par une unité d'évaluation d'un système, l'unité d'évaluation comprenant un stockage de données (42) et mettant en œuvre un modèle de classification (43) et recevant des données brutes de capteur via une liaison de communication à longue portée (7), le procédé comprenant les étapes suivantes:
a) déterminer un ensemble d'états corporels mesurables à partir d'un groupe d'états corporels d'un corps humain, les états corporels étant indicatifs d'un état de ménopause d'un corps humain,
b) déterminer un ensemble de cibles d'apprentissage, les cibles d'apprentissage étant des humains pouvant adopter un état de ménopause et ayant un état connu concernant leur état de ménopause en tant qu'informations sur l'état de ménopause,
c) mesurer, par des capteurs dont sont pourvus les cibles d'apprentissage, les états corporels mesurables de l'ensemble d'états corporels mesurables de chacune des cibles d'apprentissage pendant une période de temps prédéterminée pour fournir des informations sur l'état corporel mesuré pour chacune des cibles d'apprentissage, et détecter l'apparition d'un symptôme d'un état de ménopause,
d) prétraiter les informations sur l'état corporel mesuré pour fournir des informations prétraitées sur l'état corporel, en appliquant un filtre passe-bas, en réduisant un taux d'échantillonnage, en appliquant une fenêtre de gauche glissante ou en étiquetant des symptômes de périménopause en tant qu'informations de classification,
e) fournir le modèle de classification mis en œuvre par ordinateur, adapté pour classer un état de ménopause, c'est-à-dire, un modèle de classification formé pour classer un état de périménopause ou l'absence de celui-ci,
f) entrer les informations retraitées sur l'état corporel d'une cible d'apprentissage en tant qu'informations d'entrée d'apprentissage dans le modèle de classification,
g) entrer l'apparition détectée du symptôme d'un état de ménopause en tant qu'informations de classification d'apprentissage dans le modèle de classification,
h) adapter le modèle de classification en fonction des informations d'entrée d'apprentissage et des informations de classification d'apprentissage.

2. Procédé selon la revendication 1, dans lequel
à l'étape c), les informations sur l'état corporel mesuré sont une série chronologique d'états corporels mesurés, respectivement.

3. Procédé selon l'une quelconque des revendications 1 à 2, dans lequel
à l'étape e), le modèle de classification mis en œuvre par ordinateur est un modèle d'arbre décisionnel ou un modèle arborescent aléatoire ou un modèle de réseau neuronal artificiel, en particulier un modèle de réseau neuronal récurrent ou un modèle de réseau neuronal convolutionnel, en particulier encore un modèle de réseau d'état d'écho ou un modèle de machine à état liquide.

4. Procédé selon l'une quelconque des revendication 1 à 3, dans lequel
à l'étape a), l'ensemble d'informations sur l'état corporel contient un sous-ensemble de l'ensemble:
- l'activité électrodermique,
- la fréquence cardiaque, la VFC et la Pression Artérielle, en particulier les informations photopléthysmographiques et les informations ECG
- la température corporelle
- l'activité de mouvement du corps, en particulier la relocalisation ou l'accélération,
ou
- les informations sur l'environnement corporel, en particulier la température ambiante, l'humidité ambiante ou la pression ambiante.

5. Procédé selon l'une quelconque des revendications1 à 4,
dans lequel l'étape c) comprend en outre l'étape suivante:
ca) mesurer l'activité électrodermique et fournir des informations sur l'activité électrodermique mesurée,
et l'étape d) comprend en outre les étapes suivantes:
da) transformer les informations sur l'activité électrodermique mesurée en informations SCL et en informations SCR, et
db) fournir les informations SCL ou les informations SCR en tant qu'informations sur l'état corporel mesuré,
et l'étape f) comprend l'étape suivante:
fa) entrer les informations SCL ou les informations SCR en tant qu'informations sur l'état corporel mesuré dans le modèle de classification.

6. Procédé selon l'une quelconque des revendications1 à 5,
dans lequel l'étape h) comprend en outre l'étape suivante:
ha) fournir les informations SCL ayant le poids le plus élevé de l'ensemble d'états corporels mesurables pour la classification.

7. Procédé selon l'une quelconque des revendications 1 à 6,
dans lequel l'étape d) comprend en outre au moins l'une des étapes suivantes:
dc) appliquer un filtre passe-bas, en particulier un filtre de Butterworth, en particulier un filtre de Butterworth avec une fréquence de coupure de 0,5 Hz, aux informations sur l'état corporel mesuré,
dd) adapter un taux d'échantillonnage des informations sur l'état corporel mesuré à 1 Hz pour les états corporels mesurables de l'ensemble d'états corporels mesurables,
de) lisser les informations sur l'état corporel mesuré en appliquant une fenêtre de gauche glissante de 60 secondes, en particulier en déterminant une moyenne mobile simple en formant la moyenne non pondérée des 60 échantillons mesurés précédents,
df) fournir aux informations sur l'état corporel mesuré des informations de classification de telle sorte que les informations sur l'état corporel mesuré qui se produisent dans une période de temps de 180 secondes précédant et de 180 secondes suivant un moment de la détection d'un symptôme d'un état de ménopause soient étiquetées comme appartenant au symptôme de l'état de ménopause, et que les informations sur l'état corporel mesuré qui se produisent en dehors de cette période de temps soient étiquetées comme n'appartenant pas à un symptôme d'un état de ménopause.

8. Système mis en œuvre par ordinateur (1) permettant de classer un état de ménopause, comprenant:
une unité de capteur (2),
une unité de transmission (3), et
une unité d'évaluation (4), contenant un modèle de classification prévu selon l'une quelconque des revendications 1 à 7,
dans lequel le système est configuré pour exécuter les étapes pour une cible à diagnostiquer, la cible à diagnostiquer étant un être humain pouvant adopter un état de ménopause et ayant un état inconnu concernant son état de ménopause en tant qu'informations sur l'état de ménopause, comportant au moins un capteur au niveau du corps de la cible à diagnostiquer, à un emplacement du corps de capteur:
i) mesurer, au moyen d'au moins un capteur, un ensemble d'états corporels mesurables à partir d'un groupe d'états corporels d'un corps humain, les états corporels étant indicatifs d'un état de ménopause d'un corps humain et étant un sous-ensemble de l'ensemble d'états corporels mesurables selon l'étape a), en tant qu'informations de diagnostic de l'état corporel mesuré,
j) prétraiter - par l'unité d'évaluation - les informations de diagnostic de l'état corporel mesuré pour fournir des informations prétraitées de diagnostic de l'état corporel,
k) entrer - par l'unité d'évaluation - les informations prétraitées de diagnostic de l'état corporel de la cible à diagnostiquer en tant qu'informations d'entrée de diagnostic dans le modèle de classification,
l) classer un état de ménopause de la cible à diagnostiquer en tant qu'informations sur l'état de ménopause,
m) utiliser les informations classifiées sur l'état de ménopause pour fournir un diagnostic sur l'état de ménopause de la cible à diagnostiquer, dans lequel
l'unité de capteur est configurée pour mesurer un ensemble d'états corporels mesurables à partir d'un groupe d'états corporels d'un corps humain, les états corporels étant indicatifs d'un état de ménopause d'un corps humain et étant un sous-ensemble de l'ensemble d'états corporels mesurables, et transmettre les états corporels mesurés en tant qu'informations d'état corporel mesuré à l'unité de transmission (3),
l'unité de transmission (3) est configurée pour recevoir les informations sur l'état corporel de l'unité de capteur (2) et pour transmettre les informations sur l'état corporel à l'unité d'évaluation (4), et
l'unité de capteur (2) est configurée pour exécuter l'étape i) et l'unité d'évaluation (4) est configurée pour exécuter les étapes k) et 1).

9. Système selon la revendication 8, dans lequel
à l'étape 1), l'ensemble d'informations sur l'état corporel contient un sous-ensemble de l'ensemble:
- l'activité électrodermique,
- la fréquence cardiaque, la variabilité de la fréquence cardiaque et la pression artérielle, en particulier les informations photopléthysmographiques et les informations ECG
- la température corporelle
- l'activité de mouvement du corps, en particulier la relocalisation ou l'accélération,
ou
- les informations sur l'environnement corporel, en particulier la température ambiante, l'humidité ambiante ou la pression ambiante.

10. Système selon l'une quelconque des revendications 8 à 9,
dans lequel l'étape 1) comprend en outre l'étape suivante:
la) mesurer l'activité électrodermique et fournir des informations de diagnostic de l'activité électrodermique mesurée,
et l'étape m) comprend en outre les étapes suivantes:
ma) transformer les l'informations de diagnostic sur l'activité électrodermique mesurée en information SCL et en information SCR, et
mb) fournir les informations SCL ou les informations SCR en tant qu'informations sur l'état corporel mesuré à diagnostiquer,
et l'étape n) comprend l'étape suivante:
na) entrer les informations SCL ou les informations SCR en tant qu'informations de diagnostic de l'état corporel mesuré dans le modèle de classification,
ou l'étape o) comprend en outre l'étape suivante:
oa) prendre en considération les informations SCL ayant le poids le plus élevé de l'ensemble d'états corporels mesurables pour la classification.

11. Le système selon l'une quelconque des revendications 8 à 10,
dans lequel l'étape m) comprend en outre au moins l'une des étapes suivantes:
mc) appliquer un filtre passe-bas, en particulier un filtre de Butterworth, en particulier un filtre de Butterworth avec une fréquence de coupure de 0,5 Hz, aux informations de diagnostic de l'état corporel mesuré,
md) adapter un taux d'échantillonnage des informations de diagnostic de l'état corporel mesuré à 1 Hz pour les états corporels mesurables de l'ensemble d'états corporels mesurables,
me) lisser les informations de diagnostic de l'état corporel mesuré en appliquant une fenêtre de gauche glissante de 60 secondes, notamment en déterminant une moyenne mobile simple en formant la moyenne non pondérée des 60 échantillons mesurés précédents,
mf) appliquer un filtre supplémentaire aux informations de diagnostic de l'état corporel mesuré en fonction des informations de diagnostic de l'état corporel mesuré, ou
mg) fournir une unité de capteur à un emplacement de capteur (52), dans lequel l'emplacement de capteur (52) est disposé au niveau d'un corps humain, en particulier au niveau d'un torse, en particulier au niveau de la partie inférieure du thorax, en particulier au-dessous du sternum, soit au centre, soit sur le côté frontal gauche du thorax de la cible à diagnostiquer.

12. Système selon l'une quelconque des revendications 8-11, dans lequel l'unité de capteur (2), l'unité de transmission (3) ou l'unité d'évaluation (4) est configurée pour exécuter l'étape m).

13. Système selon l'une quelconque des revendications 8 à 12, dans lequel l'unité de capteur (2) comprend en outre
une partie capteur (22) sur un côté proximal de l'unité de capteur (2), la partie capteur comprenant une forme convexe, et
une partie de poussée (21) qui est configurée pour pousser la partie capteur vers une surface cutanée (51) à un emplacement de capteur (52), de telle sorte que la partie capteur indente la surface cutanée.
